# EUROPEAN PATENT SPECIFICATION

(11) **EP 0 507 904 B1**
(45) Date of publication and mention of the grant of the patent: **20.01.1999**
(21) Application number: 91917738.6
(22) Date of filing: 25.10.1991
(51) Int. Cl.: C12Q 1/68

(54) **METHOD FOR EVALUATING THE ADEQUACY OF CLINICAL SPECIMENS FOR HYBRIDIZATION ASSAYS AND KIT FOR PERFORMING THE EVALUATION**
VERFAHREN ZUR BEWERTUNG DER TAUGLICHKEIT VON KLINISCHEN PROBEN FÜR HYBRIDISIERUNGSTESTS UND EIN DAFÜR ANGEPASTES KIT
PROCEDE SERVANT A EVALUER SI DES EPROUVETTES CLINIQUES CONVIENNENT A DES ANALYSES D'HYBRIDATION ET KIT SERVANT A EFFECTUER L'EVALUATION

(30) Priority: 26.10.1990 US 604575
(43) Date of publication of application: 14.10.1992
(73) Proprietor: SANGTEC MEDICAL AB, 161 02 Bromma (SE)
(72) Inventor: KALLIO, Arja, SF-02160 Espoo (FI); JALAVA, Tarja, SF-00420 Helsinki (FI)
(74) Representative: Fagerlin, Heléne
(86) International application number: FI9100321
(87) International publication number: WO9207955

(56) References cited:
- EP-A- 0 314 293
- SAMBROOK, FRITSCH & MANIATIS 'MOLECULAR CLONING (LABORATORY MANUAL)' 1989 , COLD SPRING HARBOUR PRESS , US see page 6.15 see page 6.44 see page E.5
- GYNECOLOGIC ONCOLOGY vol. 30, 1988, NY, US pages 251 - 264; S. PARKKINEN ET AL: 'SCREENING OF PREMALIGNANT CERVICAL LESIONS FOR HPV 16 DNA BY SANDWICH AND IN SITU HYBRIDIZATION TECHNIQUES' cited in the application see the whole document
- AM J OBSTET GYNECOL vol. 160, no. 2, February 1989, ST. LOUIS, MISSOURI, US pages 304 - 308; S. H. VERMUND ET AL: 'MOLECULAR DIAGNOSIS OF GENITAL HUMAN PAPPILLOMAVIRUS INFECTION: COMPARISON OF TWO METHODS USED TO COLLECT EXFOLIATED CERVICAL CELLS' cited in the application SA 52525 030

## Description

### TECHNICAL FIELD

The present invention relates to hybridization tests for infectious disease diagnostics and more specifically to methods and reagents for evaluating specimen adequacy for detecting and identifying nucleic acids in clinical samples.

### BACKGROUND OF THE INVENTION

Diagnostic methods based on nucleic acid hybridization techniques are becoming increasingly more important in clinical diagnostics. Especially the diagnosis of infectious diseases has benefitted from the use of hybridization based methods. For viruses that are infective without expressing viral proteins or are incorporated into the genome of the host cell, nucleic acid hybridization is the only diagnostic method possible.

Human papillomaviruses, which are implicated as an etiologic agent in the delevopment of neoplasia and invasive carcinoma of the cervix, are such a diagnostic problem because they cannot be cultivated in vitro and viral antigens are not reliably detected in the specimens. Human papillomavirus infections of the cervix are detected from clinical samples, i.e. cells collected as exocervical spatula scrapes or endocervical swabs or brushes, analogous to Papanicolaou smear collection. However, inadequate cell recovery is common, leading to false negative results in the hybridization assay.

The reliance of the Papanicolaou smear and the problems created by inadequate cell recovery has long been recognized in the field of cytopathology, where large efforts have been made to increase the sample collection methods. See e.g. Greening (Diagnostic Cytopathology, Vol 1, No. 1, 1985) and Yobs et al. (Obstetrics & Gynecology, Vol. 65, No. 2, 1985).

In molecular diagnosis, i.e. hybridization assays, the problem of the sample accuracy is even more acute than in cytological tests, because there is no microscopic evaluation of the samples in hybridization assays, except in situ hybridization assays. Vermund et al. (Am J Obstet Gynecol, Vol. 160, No. 2, 1989) describe an improved method of collecting cellular samples for molecular diagnosis of human papillomavirus. This reference points out the importance of sample accuracy in screening HPV infections.

In a study performed in Finland (Parkkinen et al. Gynecologic Oncology, 30, 251-264, 1988) the sensitivity and applicability of sandwich hybridization and in situ hybridization tests for routine diagnosis of cervical HPV infections from smears was tested. The study confirmed the importance of the quality of the specimen. Because of the difficulties in evaluating the adequacy of the scrapes Parkkinen et al. included an internal standard into the sandwich assay to measure the actual cell number in the specimen. The estimation of the cell number was performed by a parallel hybridization test using sandwich hybridization reagents for the human pro-alpha2(I)-collagen gene, which is a single-copy gene. The inclusion of this internal standard suggested that a minimum of 500,000 cells are required for an adequate scrape specimen and Parkkinen et al. conclude that a new specimen should be collected if the internal standard shows less cells than 500,000. The drawback of this method is, that the evaluation of the adequacy of the specimen is not available until the next day. In the case of an inadequate sample the patient thus has to be re-called for a new test, which might be very inconvenient.

Even if the test was to be performed using a sandwich format in solution, such as the AffiProbe™ HPV test, commercially available from Orion Corporation, where the results are obtained during one day, the time-lag between the specimen collection and the result of the adequacy of the taken specimen is too long (3 - 5 hours).

Accordingly, there is an established need for a method for evaluating the accuracy of the collected clinical specimens prior to hybridization. Further, such a method should be easy to use without special equipment requirements. The results of such a test should be available within a short time period, preferably while the patient is waiting in the clinic.

### SUMMARY OF THE INVENTION

It is the object of this invention to provide a fast and reliable method for evaluating the adequacy of a clinical specimen, and to provide a reagent kit for performing this method. The result is visible to the eye and easy to interpret. The specimen adequacy evaluation kit according to this invention is a solution to a long felt need in the clinical diagnostics.

The method is based on the use of chemical agents which intercalate to double stranded DNA molecules usually by insertion between the base pairs. This causes the spreading of adjacent base pairs leading to an increase in molecular length of the duplex. A wide variety of intercalating agents can be used in present invention. The intercalating agent are chosen from a group consisting of chemical substances that are either directly visually detectable after intercalation or indirectly visually detectable by the addition of an detectable moiety to the intercalator.

In a preferred mode the kit for specimen evaluation according to the present invention comprises a 1% agarose gel containing EtBr (670 ng/ml) casted on a support to facilitate the handling of the gel.

A representative sample of the clinical specimen is applied onto the agarose gel and comparative DNA standards are added to the plate. The adequacy evaluation can be performed immediately after the samples have been allowed to absorb into the gel. The evaluation is performed visually and the specimen is regarded as accurate when the intensity of the signal from the adequately diluted sample equals that of the corresponding standard signal.

### BRIEF DESCRIPTION OF DRAWINGS

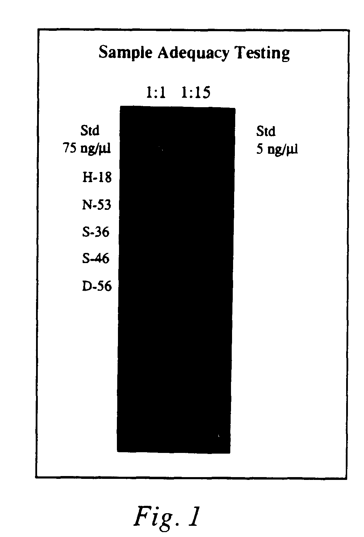

Figure 1 is a photograph of a test slide taken under UV light. The top dots on the slide correspond to standard HPV-DNA (75 ng/µl and 5 ng/µl). The abbreviations M-18, N-53, S-36, S-46 and D-56 denote five different clinical samples, undiluted in the left lane and diluted 1:15 in the right lane.

Figure 2 is a drawing of a solid support for the gel and its package.

### DETAILED DESCRIPTION OF THE INVENTION

The present invention resides in a method for easy determination of whether a clinical specimen contains a sufficient amount of cells to be reliably used in a hybridization test. The term "clinical specimen" as used herein refers to any clinical specimen containing cellular material. The clinical specimen is usually a cellular scrape, obtained by conventional methods used in cytopathology for e.g. PAP-smears. The method and the kit used to perform the test is, however, applicable to all cellular samples, such as scrapes, punches, lavages, body fluids.

The method according to the invention is based on the use of chemical agents that bind specifically to nucleic acids, creating visually detectable signals. Especially useful nucleic acid staining agents are nucleic acid intercalators, which interact with the normal helix structure of double stranded nucleic acids by non-covalent insertion between the base pairs. Suitable nucleic acid staining agents to be used in the present invention are agents that are directly or indirectly visually detectable after interacting with nucleic acids. Such compounds are exemplified by e.g phenantridines, acridines, phenazines, coumarines, flavanoids, quinolines and the like.

Preferably the intercalating agent is such that it is easily visualized by fluorescence or by a color reaction. A preferred agent is ethidium bromide (EtBr) which intercalates between the stacked bases of DNA. The fixed position of this group and its close proximity to the bases causes dye bound to DNA to display an increased fluorescent yield compared to free dye. The visual detection is based on UV-induced fluorescence emitted by EtBr molecules intercalated into the nucleic acid molecules.

Other suitable nucleic acid staining agents are the commercially available fluorescent dye Hoechst H33258, flavanoids like quercetin, kaempterol and isorhamnetin and coumaines like bergapten, angelicin and herniarin.

The present invention is further based on a plate assay modified for crude samples containing whole cells. The use of EtBr-agarose plates was originally described for quantification of purified DNA (Maniatis et al., Molecular Cloning, A Laboratory Manual, p 468-469, 1982). In this laboratory method the purified DNA sample is spotted onto the surface of a 1% agarose slab containing 0.5 µg/ml of ethidium bromide. The gel is then allowed to stand at room temperature for a few hours to allow small contaminating molecules to diffuse away, whereafter the gel is photographed using short-wavelength UV illumination. The DNA concentration of the sample is then estimated by comparing the intensity of fluorescence in the sample with that of standard solutions. A similar method is disclosed in Sambrook et al., Molecular cloning, p E.5-E.7, 1989.

It has now surprisingly been found that this method can be used on crude samples containing whole cells, without any treatment to destroy the cells or any purification steps.

A suitable gel containing the intercalating agent for use in the present invention is any gel onto which the sample can applied and which does not disturb the visualization the intercalating agent and which can be attached to a solid support. Preferred gels are e.g. agarose gels and polyacrylamide gels. The solid content of the gel should not be too stiff, as this might hamper the absorption of the sample. On the other hand a too soft gel is vulnerable to mechanical damage. A suitable gel is a 0.5 - 5% agarose gel, preferable 0.8 - 2% and most preferable a 1% agarose gel, containing a suitable amount of intercalator, which can be cast on plastic supports. An other preferred gel is polyacrylamide cross linked with bis acrylamide, which has the further advantage that it can be cross linked to stick to a glass support. Preferred amounts of intercalators depends on the type of intercalator used. The amount has to be optimized so that the fluorescence of the intercalator does not disturb the interpretation of the signal from the sample dots. In the preferred mode where the intercalator is ethidium bromide, a preferred amount is 0.3 - 1 µg/ml. The agarose gel is made in an nearly neutral aqueous solution, such as E-buffer (0.04 M Tris-acetate, 0.002 M EDTA). The agarose-buffer mixture is heated to dissolve the agarose and thereafter cooled to about +50°C. The intercalator is added into the cooled agarose gel mixture. A suitable amount of this mixture is cast on a solid support and allowed to cool at room temperature. The ready-made plates are stored at +4°C to inhibit drying of the gel. Optionally an antibacterial agent, such as sodium azide, might be included to prevent bacterial contamination during storage.

To facilitate the handling of the gel it is cast onto a solid support. Preferably the solid support is a glass or plastic plate, such as polystyrene or any other polymeric support, which allows agarose or polyacrylamide gel to adhere. The plate is preferable provided with a cover or coating, preferably a container that protects the intercalator containing plate from mechanical damage, drying and illumination. The ready-to-use gel is provided in a packaged kit format, including all reagents needed for the test, such as diluents and positive standard reagents.

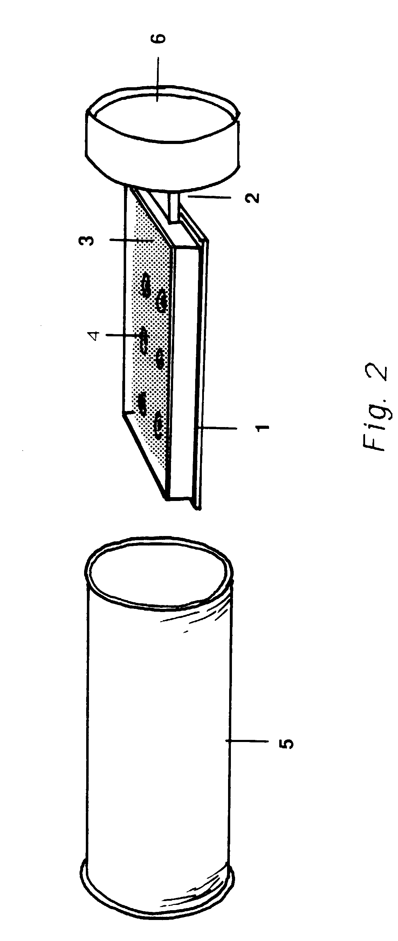

Figure 2 shows a preferred plate device where the solid support is a polystyrene tray provided with edges (1) to hold the gel (3) and a handle (2) to facilitate handling of the gel as presented in Figure 2. The gel supporting plate is preferably packed in a container (5) provided with a cap (6) to prevent drying.

The object of the present invention is to provide a convenient method for determining the nucleic acid content of a crude clinical sample containing whole cell. Therefore a main effort was made to determine the sensitivity of the test, i.e. whether the amount of cells in an adequate specimen is clearly visible in the test, and to determine suitable standards to be included in the test. These determinations were made for clinical specimens, cellular scrapes, to be used in a hybridization test for detecting and identifying human papillomavirus, the AffiProbe™ HPV kit (Orion Pharmaceutica), which is based on a solution hybridization method disclosed in British Patent No. 2,169,403. It will, however, easily be anticipated by one skilled in the art, that the method and test kit according to the present invention is equally applicable to any clinical specimens for use in any hybridization test.

In the method according to the present invention a small sample, one to a few microliters, of the clinical specimen is spotted directly on the gel for visualization. The volume of the sample to be tested is determined by the sensitivity of the hybridization test to be used. In case the clinical specimen is very dilute, i.e. is taken into a large volume of diluent, it might be convenient to centrifuge the clinical cell specimen to provide a cell pellet. The centrifuged pellet is then re suspended in a suitable volume, e.g. 100 µl of a suitable diluent solution, to form a more concentrated specimen. The optimal dilution is dependent of the type of clinical specimen used and the molecular diagnostic test to be performed, e.g. detection of a specific pathogenic microorganism. A suitable volume of this sample suspension, preferably a one to a few microliters, is pipetted onto the gel plate together with suitable DNA control solutions containing clearly visible amounts of a standard DNA. The amounts of standard DNA in the control samples are experimentally determined so that an undiluted DNA control sample provides a strong visible signal, whereas the diluted DNA control gives a dim, but clearly visible signal. In a most preferred embodiment of the present invention the DNA control is a solution containing 75 ng of DNA per µl (high positive control) and a dilution of this standard containing 5 ng of DNA per µl (low positive control). The applied samples are allowed to drain into the agarose gel for a few minutes.

The samples should be analysed and the adequacy of the specimen evaluated by examination in a suitable illumination, preferably during the same day. The clinical test specimens are regarded as containing a sufficient amount of cells if the intensity of the signal of the diluted specimen derived sample dot is at least equal to that of the low-positive control dot. A clinical specimen thus evaluated is regarded as an adequate specimen for the hybridization test.

The application of the test sample and the standard reagents may be facilitated by providing the gel with marked sample application spots, e.g. punched depressions (4) in the gel as in Figure 2.

In the following a preferred embodiment of the present invention is more specifically described in examples illustrating a test for determining the adequacy of clinical specimens for use in the detection and identification of human papillomavirus by AffiProbe™ (Orion Pharmaceutica). These examples are not intended to limit the scope of this invention. Different formats of the solid support and the applicability of the test for other infectious organisms are easily anticipated by one skilled in the art.

### Example 1

### Preparation of the agarose gel on a solid support

An agarose gel plate containing ethidium bromide (EtBr) was made by adding 1.5 g of agarose into 150 ml of E-buffer (0.04 M Tris-acetate, 0.002 M EDTA, pH 8). The mixture was autoclaved for 20 min and cooled in +50°C water bath. 10 µl of EtBr (10 mg/ml) was added into the cooled agarose gel mixture. Then 7 ml of this mixture was pipetted onto a 13 cm² plastic support and allowed to cool at room temperature. The EtBr-agarose gel plates were stored at +4°C in containers impervious to light.

### Example 2

### Determination of the amount of cells visible in UV-illumination

According to Parkkinen et al. (supra) an adequate scrape specimen should contain a minimum of 500,000 cells to be detectable in a sandwich hybridization test. The hybridization test AffiProbe™ HPV is performed on a 30 µl sample drawn from the clinical scrape specimen. This example was performed to clarify whether it is possible to recognize nucleic acids from a cellular sample containing 500,000 cells per 30 µl on a EtBr-agarose gel plate without any extraction, purification or concentrating steps.

Cultured human R1610 cells were counted in a Coulter counter and 5x10⁵ cells was suspended in 30 µl of PBS-E buffer and 1 µl of this suspension (1.7x10⁴ cells) was spotted onto an agarose plate together with a dilution series of the same sample, as shown in Table 1. The fluorescence of the spots was compared to standard spots containing purified DNA in known amounts.

5 ng of a pure DNA standard was clearly visible on the plate. The R1610 cell suspension containing 1.7x10³ cells/µl gave a stronger fluorescence than the 5 ng spot of pure DNA.

These results suggest that a suitable volume (1 µl) of a cell suspension containing 5x10⁵ cells / 30 µl of DNA is clearly visible without releasing or purifying the DNA content of the cells.

**Table I**

| Amount of cells R1610 | Evaluation (visible spot) | Standard DNA (ng/spot) | Evaluation (visible spot) |
|---|---|---|---|
| 1.7 x 10⁴ | + | 100 | + |
| 1.7 x 10³ | + | 10 | + |
| 1.7 x 10² | - | 5 | + |
| 1.7 x 10 | - | 1 | - |

### Example 3

### Determination of the appropriate dilution of clinical specimens.

The cell contents of four clinical cell specimens, cervical scrapes collected for the AffiProbe™ HPV test, were counted in a Coulter counter and a dilution series of the samples was spotted onto an EtBr-agarose plate, whereafter the spots were visually analysed in UV light. The result of the test is shown in Table II.

The sensitivity of the AffiProbe™ test requires samples containing 300.000 - 400.000 cells in 30 µl of PBS-E -buffer for testing the presense of human papillomavirus.

It was theoretically calculated that the 1:15 dilution of the AffiProbe sample which gave approximately the same fluorescence as 5 ng of pure DNA, contained about 2.3 ng of DNA. (Calculation based on the approximation that 10⁶ cells contain 3µg of DNA). The stronger fluorescence compared to pure DNA is possibly due to cellular RNA. As one of the samples clearly contained blood, it was also clarified that blood-stained specimens are equally well tolerated in the test according to the present invention. This was further confirmed in the tests disclosed in Examples 4-6.

As the four specimens tested in this example were verified to contain enough cells, the conclusion of this test is that a clinical specimen contains enough cells for further testing by AffiProbe™, if 1 µl of a 100 µl clinical specimen gives a positive signal at a 1:15 dilution.

**Table II**

| Cells/specimen (30 µl) | Dilution | Cells/spot (1 µl) | Evaluation of results |
|---|---|---|---|
| 3.4x10⁵ | 1:1 | 1.13x10⁴ | + |
| | 1:2 | 5.67x10³ | + |
| | 1:5 | 2.27x10³ | + |
| | 1:10 | 1.13x10³ | + |
| | 1:15 | 7.56x10² | + |
| | 1:20 | 5.67x10² | - |
| | 1:40 | 2.83x10² | - |
| | 1:80 | 1.41x10² | - |
| | | | |
| 4.0x10⁵ | 1:1 | 1.33x10⁴ | + |
| | 1:2 | 6.67x10³ | + |
| | 1:5 | 2.67x10³ | + |
| | 1:10 | 1.33x10³ | + |
| | 1:15 | 7.80x10² | + |
| | 1:20 | 6.67x10² | + |
| | 1:40 | 3.34x10² | - |
| | 1:80 | 1.67x10² | - |
| | | | |
| 4.2x10⁵ | 1:1 | 1.40x10⁴ | + |
| | 1:2 | 7.00x10³ | - |
| | 1:5 | 2.80x10³ | - |
| | 1:10 | 1.40x10³ | + |
| | 1:15 | 9.33x10² | + |
| | 1:20 | 7.00x10² | + |
| | 1:40 | 3.50x10² | - |
| | 1:80 | 1.75x10² | - |
| | | | |
| 4.3x10⁵ | 1:1 | 1.43x10⁴ | - |
| | 1:2 | 7.17x10³ | + |
| | 1:5 | 2.87x10³ | + |
| | 1:10 | 1.43x10³ | + |
| | 1:15 | 9.56x10² | + |
| | 1:20 | 7.17x10² | + |
| | 1:40 | 3.58x10² | - |
| | 1:80 | 1.79x10² | - |

### Example 4

### Sample adequacy evalution of cervical scrapes for HVP testing

The applicability of the specimen adequacy evaluation method was further determined by testing clinical specimens from a Scandinavian multicenter study in which cervical scrapes were collected from patients susceptible for papilloma virus infection. All clinical specimens were subjected to visual inspection of the cell content. This visual inspection was done by centrifuging the clinical specimens, whereafter the cell content was evaluated from the size of the obtained cell pellet. If the cell pellet was very small or absent the specimen was regarded inadequate. The presence of the virus was tested by the AffiProbe™ HPV test and other conventional HPV detection tests.

Out of 420 collected scrapes a total of 48 specimens were discarded from the study because the visual inspection of the specimen indicated that inadequacy. This large percentage (11%) shows the real need for a screening method for determining the specimen adequacy at the clinic.

The result of the visual inspection was confirmed by the test according to the present invention. The specimens were diluted as shown in Table III and spotted onto an EtBr-agarose plate. DNA standards containing 75 ng/µl and 5 ng/µl were also spotted onto the plate.

**Table III**

| Specimen code | Dilution | | |
|---|---|---|---|
| | 1:1 | 1:10 | 1:15 |
| A28 | - | - | - |
| A29 | + | + | - |
| A37 | - | - | - |
| A82 | - | - | - |
| A94 | + | - | - |
| B43 | + | - | - |
| B45 | - | - | - |
| B47 | - | - | - |
| B48 | - | - | - |
| B51 | - | - | - |
| B58 | - | - | - |
| B67 | - | - | - |
| C10 | + | - | - |
| C28 | + | - | - |
| C42 | - | - | - |
| C43 | - | - | - |
| C50 | - | - | - |
| C58 | + | - | - |
| C59 | - | - | - |
| C60 | + | - | - |
| D02 | - | - | - |
| D46 | - | - | - |
| D54 | + | - | - |
| D62 | - | - | - |
| D66 | + | - | - |
| D82 | - | - | - |
| E10 | - | - | - |
| E11 | - | - | - |
| E16 | + | - | - |
| E20 | + | - | - |
| E21 | + | - | - |
| E23 | - | - | - |
| E24 | - | - | - |
| E25 | - | - | - |
| E29 | - | - | - |
| E31 | + | - | - |
| E32 | + | - | - |
| E41 | - | - | - |
| E42 | - | - | - |
| E43 | + | - | - |
| E45 | + | + | - |
| E54 | - | - | - |
| E57 | - | - | - |
| E58 | - | - | - |
| E65 | - | - | - |
| E68 | + | - | - |
| E69 | + | - | - |
| E72 | + | - | - |

### Example 5

### Sample adequacy evalution of doubtful specimens

Two specimens from the multi-center study described in Example 4, which yielded discrepant results in the visual interpretation of specimen adequacy (visual inspection was regarded adequate by two persons, inadequate by one), were also tested for specimen adequacy.

The specimens were diluted and spotted onto an EtBr-agarose plates as described in Example 2. The results shown in Table IV verified that both specimens contained a sufficient amount of cells.

**Table IV**

| Specimen code | Dilution | | | |
|---|---|---|---|---|
| | 1:1 | 1:5 | 1:10 | 1:15 |
| S1 | + | + | + | + |
| S2 | + | + | + | + |

### Example 6

### Sample adequacy evalution of specimens regarded as adequate by visual inspection

A randomized sample (n=33) of the specimens included in the multi-center study described in Example 4, which were interpreted as accurate for the hybridization test by visual inspection, were tested for specimen accuracy by the method according to the present invention. This test showed that only 67% of the specimens contained a sufficient amount of cells, whereas 33% of the clinical specimens were not adequate for a diagnostic hybridization test.

The specimens tested are listed in Table V. The different specimen codes (A-E) indicate that the clinical specimens were collected at different medical centers.

Out of the 11 inadequate specimens only one gave a positive signal in the AffiProbe™ test. In two cases it was verified by further testing that the patient really was infected by HPV, whereas the remaining 8 specimens were not verified.

**Table V**

| Specimen code | Dilution | | | Accurate % |
|---|---|---|---|---|
| | 1:1 | 1:10 | 1:15 | |
| A10 | ++ | | + | |
| A20 | ++ | | + | |
| A32 | ++ | | + | |
| A43 | + | | - | |
| A52 | + | | - | |
| A63 | ++ | | + | |
| A73 | ++ | | + | |
| A84 | + | | + | |
| A91 | ++ | | + | |
| A100 | ++ | | + | 80 |
| | | | | |
| B18 | - | | - | |
| B29 | + | | - | |
| B39 | ++ | | + | |
| B54 | + | | - | |
| B65 | + | | - | 20 |
| | | | | |
| C03 | ++ | | + | |
| C15 | ++ | | + | |
| C25 | ++ | | + | |
| C36 | + | | - | |
| C46 | ++ | | + | |
| C52 | ++ | | + | 83 |
| | | | | |
| D11 | + | | + | |
| D21 | ++ | | + | |
| D28 | + | | - | |
| D42 | ++ | | + | |
| D53 | + | | - | |
| D65 | + | | - | |
| D76 | + | | + | 57 |
| | | | | |
| E12 | ++ | | + | |
| E28 | + | | + | |
| E46 | - | | - | |
| E56 | ++ | | + | |
| E74 | ++ | | + | 80 |

## Claims

1. A method for evaluating the adequacy of clinical specimens for nucleic acid hybridizaton tests comprising the steps of
(a) taking a representative sample from the specimen;
(b) applying the sample, without intermediate treatment, to a gel having dispersed therein a nucleic acid staining agent:
(c) visualizing the amount of said staining agent bound to the sample;
and
(d) comparing the amount of said staining agent bound to the sample to a known concentration of deoxyribonucleic acid and staining agent in order to determine the approximate number of cells in the sample, wherein none of the cells in said sample, which number is to be estimated, are treated to be destroyed nor have been purified.

2. The method according to claim 1, wherein the gel is selected from a group containing agarose or polyacrylamide gels.

3. The method according to claim 1, wherein the nucleic acid staining agent is an intercalating agent visually detectable when intercalated into nucleic acids.

4. The method according to claim 3, wherein the nucleic acid staining agent is ethidium bromide.

5. The method according to claim 4, wherein the nucleic acid content of the sample is evaluated by the UV-induced fluorescence emitted by ethidium bromide molecules intercalated into the nucleic acid of the sample.

6. The method according to claim 2, wherein the gel is casted on a solid support.

7. The method according to claim 6, wherein the solid support is a plastic plate.

8. Use of a kit for evaluating the adequacy of clinical specimens for nucleic acid hybridization tests in the method according to anyone of claims 1-7, said kit comprising a gel on a solid support, said gel containing an agent that specifically binds to nucleic acids creating a visually detectable signal.

9. Use according to claim 8, wherein the nucleic acid staining agent is ethidium bromide.

10. Use according to claim 8, wherein the solid support is selected from a group containing glass and polystyrene.

11. Use according to claim 8, wherein the solid support comprising the gel is packed in a UV-light impermeable protective vial.

12. Use according to claim 8, for evaluating the adequacy of clinical specimens obtained from the genital area for testing the presence of human papillomavirus by nucleic acid hybridization, wherein said kit comprises an agarose gel on a solid support, said agarose gel containing an agent that intercalates into nucleic acids creating a visually detectable signal, standard DNA solutions as positive controls and suitable diluents.

13. Use according to claim 12, wherein the intercalating agent is ethidium bromide.

## Patentansprüche

1. Verfahren zur Beurteilung der Brauchbarkeit von klinischem Untersuchungsmaterial für Nucleinsäurehybridisierungstests, umfassend die folgenden Schritte:
(a) Gewinnung einer repräsentativen Probe von dem Untersuchungsmaterial;
(b) Aufbringen der Probe ohne Zwischenbehandlung auf ein Gel, in dem ein Nucleinsäurefärbemittel dispergiert ist;
(c) Sichtbarmachen der Menge des Färbemittels, das an die Probe gebunden ist; und
(d) Vergleich der Menge des an die Probe gebundenen Färbemittels mit einer bekannten Konzentration an Desoxyribonucleinsäure und Färbemittel, um die ungefähre Zahl von Zellen in der Probe zu bestimmen, wobei die Zellen in der Probe, deren Zahl zu bestimmen ist, weder behandelt wurden, um sie aufzubrechen, noch gereinigt wurden.

2. Verfahren nach Anspruch 1, wobei das Gel ausgewählt ist aus Agarose- oder Polyacrylamidgelen.

3. Verfahren nach Anspruch 1, wobei das Nucleinsäurefärbemittel ein interkalierendes Mittel ist, das visuell nachweisbar ist, wenn es in Nucleinsäuren eingelagert ist.

4. Verfahren nach Anspruch 3, wobei das Nucleinsäurefärbemittel Ethidiumbromid ist.

5. Verfahren nach Anspruch 4, wobei der Nucleinsäuregehalt der Probe durch die UV-induzierte Fluoreszenz bestimmt wird, die von Ethidiumbromid-Molekülen emittiert wird, die in die Nucleinsäure der Probe eingelagert sind.

6. Verfahren nach Anspruch 2, wobei das Gel auf einen festen Träger gegossen ist.

7. Verfahren nach Anspruch 6, wobei der feste Träger eine Kunststoffplatte ist.

8. Verwendung eines Kits zur Bestimmung der Brauchbarkeit von klinischem Untersuchungsmaterial für Nucleinsäurehybridisierungstests in dem Verfahren nach einem der Ansprüche 1 bis 7, wobei das Kit ein Gel auf einem festen Träger umfaßt, wobei das Gel ein Mittel enthält, das spezifisch an Nucleinsäure bindet und ein visuell nachweisbares Signal erzeugt.

9. Verwendung nach Anspruch 8, wobei das Nucleinsäurefärbemittel Ethidiumbromid ist.

10. Verwendung nach Anspruch 8, wobei der feste Träger ausgewählt ist aus Glas und Polystyrol.

11. Verwendung nach Anspruch 8, wobei der das Gel umfassende feste Träger sich in einem für UV-Licht undurchdringlichen Schutzgefäß befindet.

12. Verwendung nach Anspruch 8, zur Bestimmung der Brauchbarkeit von aus dem Genitalbereich erhaltenen Untersuchungsmaterial zum Testen auf die Gegenwart von menschlichen Papillomavirus durch Nucleinsäurehybridisierung, wobei das Kit ein Agarosegel auf einem festen Träger umfaßt, wobei das Agarosegel ein Mittel enthält, das sich in Nucleinsäuren einlagert und ein visuell nachweisbares Signal erzeugt, und Standard-DNA-Lösungen als Positivkontrolle sowie geeignete Verdünnungsmittel.

13. Verwendung nach Anspruch 12, wobei das interkalierende Mittel Ethidiumbromid ist.

## Revendications

1. Procédé pour évaluer l'adéquation d'échantillons cliniques à à des essais d'hybridation d'acides nucléiques comprenant les étapes consistant
(a) à prendre un échantillon représentatif du prélèvement,
(b) à appliquer l'échantillon, sans traitement intermédiaire, sur un gel dans lequel est dispersé un agent de coloration d'acide nucléique,
(c) à visualiser la quantité dudit agent de coloration fixé à l'échantillon, et
(d) à comparer la quantité dudit agent de coloration lié à l'échantillon avec une concentration connue d'acide désoxyribonucléique et d'agent colorant afin de déterminer le nombre approximatif de cellules dans l'échantillon, aucune des cellules, dont on souhaite évaluer le nombre dans ledit échantillon, ayant ainsi subi un traitement destructeur ni une purification.

2. Procédé selon la revendication 1, dans lequel le gel est choisi parmi les gels d'agarose et les gels de polyacrylamide.

3. Procédé selon la revendication 1, dans lequel l'agent de coloration d'acide nucléique est un agent d'intercalation qui peut être détecté visuellement lorsqu'il s'intercale entre les acides nucléiques.

4. Procédé selon la revendication 3, dans lequel l'agent de coloration d'acide nucléique est le bromure d'éthidium.

5. Procédé selon la revendication 4, dans lequel la teneur en acide nucléique dans l'échantillon est évaluée par fluorescence induite par UV émise par des molécules de bromure d'éthidium intercalées dans l'acide nucléique de l'échantillon.

6. Procédé selon la revendication 2, dans lequel le gel est coulé sur un support solide.

7. Procédé selon la revendication 6, dans lequel le support solide est une plaque en matière plastique.

8. Utilisation d'un kit pour l'évaluation de l'adéquation d'un prélèvement clinique à des essais d'hybridation d'acide nucléique selon le procédé conforme à l'une quelconque des revendications 1 à 7, ledit kit comprenant un gel sur un support solide, ledit gel contenant un agent qui se lie spécifiquement à l'acide nucléique produisant ainsi un signal que l'on peut détecter visuellement.

9. Utilisation selon la revendication 8, dans laquelle l'agent de coloration d'acide nucléique est le bromure d'éthidium.

10. Utilisation selon la revendication 8, dans laquelle le support solide est choisi dans le groupe formé par le verre et le polystyrène.

11. Utilisation selon la revendication 8, dans laquelle le support solide portant le gel est-emballé dans un flacon protecteur impérméable au rayonnement UV.

12. Utilisation selon la revendication 8, pour évaluer l'adéquation de prélèvements cliniques obtenus à partir de la zone génitale afin détecter la présence de papillomavirus par hybridation d'acide nucléique, ledit kit comprenant un gel d'agarose sur un support solide, ledit gel d'agarose contenant un agent capable de s'intercaler entre les acides nucléiques en produisant un signal visible, des solutions d'ADN de référence comme témoins positifs et des diluants appropriés.

13. Utilisation selon la revendication 12, où l'agent d'intercalation est le bromure d'éthidium.
